# EUROPEAN PATENT APPLICATION

(11) **EP 4 825 304 A1**
(43) Date of publication of application: **21.10.2026**
(21) Application number: 24903546.0
(22) Date of filing: 02.12.2024
(51) Int. Cl.: A61B 1/06, A61B 1/00

(54) **ELECTRONIC ENDOSCOPE SYSTEM**

(30) Priority: 13.12.2023 JP 2023210077
(71) Applicant: HOYA CORPORATION, Shinjuku-ku Tokyo 160-8347 (JP)
(72) Inventor: SHIMOTASHIRO Shinya, Tokyo 160-8347 (JP)
(74) Representative: Lucke, Andreas
(86) International application number: PCT/JP2024/042570
(87) International publication number: WO 2025/126888

(57) **Abstract**

One aspect of the present invention is an electronic endoscopic system that includes a processor and a light source module having a light emitting device. The light source module includes a first storage device that stores a reference light amount value of the light emitting device under a predetermined condition, the reference light amount value being obtained by the light source module alone. The processor includes a second storage device and a control unit. The control unit writes a reference light amount value read from the first storage device into the second storage device after the light source module is incorporated, and at the time of system activation, compares an activation-time light amount value, which is the reference light amount value of the light emitting device acquired under the predetermined condition, with each of a first light amount value, which is the reference light amount value read from the first storage device, and a second light amount value, which is the reference light amount value read from the second storage device, and determines which of the data in the first storage device and the second storage device is more probable data on a basis of a comparison result.

## Description

### Technical Field

The present invention relates to an electronic endoscopic system configured to perform imaging of a body tissue.

### Background Art

In a medical device field, there is known an endoscopic system capable of generating an image suitable for diagnosing a lesion hidden in a body lumen, by illuminating a body tissue in the body lumen and imaging the illuminated body tissue in the body lumen as an object. Conventionally, a lamp light source such as a Xenon lamp or a halogen lamp that emits a white-color light has been used as an illumination light. However, recently, a light source module including a light emitting diode (LED) that emits light having a specific wavelength band has been used instead of the lamp light source (for example, WO 2016/056476 A).

Since the output characteristic of the emitted light from the light source is generally nonlinear, the output characteristic of the light source is measured in advance by a calibration device, and a correction coefficient for correcting the output characteristic to be linear is recorded in a nonvolatile memory. When the light source module is used, the output characteristic of the emitted light is corrected to be linear using the recorded correction coefficient.

### Summary of Invention

### Technical Problem

In a case where a highly durable storage device (memory) is used as a nonvolatile memory that stores the correction coefficient for correcting the output characteristic of the light source, that will lead to an increase in the cost of the system, which is not favorable. On the other hand, in a case where the data recorded in the storage device is lost because the highly durable storage device has not been used, it is necessary to determine the correction coefficient by measuring the output characteristic of the light source again using the calibration device. However, since many man-hours are required for the measurement, it is troublesome to perform the calibration work again. Even in a case where the light source module itself is replaced, the calibration work for a new light source module is required, which is also troublesome.

Therefore, an object of the present invention is to ensure robustness of data without using a highly durable storage device in a case where data for correcting an output characteristic of a light source is stored in a storage device in an electronic endoscopic system.

### Solution to Problem

One aspect of the present disclosure is an electronic endoscopic system configured to perform imaging of a body tissue, and includes
a processor that performs processing of a captured image of the body tissue, and
a light source module that is incorporable into the processor for an electronic endoscope and generates illumination light for the body tissue on the basis of light emitted from at least one light emitting device.

The light source module includes a first storage device that stores a correction coefficient for correcting an output characteristic of the light emitting device and a reference light amount value of the light emitting device under a predetermined condition, the correction coefficient and the reference light amount value being obtained before the light source module is incorporated into the processor.

The processor includes a second storage device, a photodetector that detects a light amount of the light emitting device, and a control unit.

The control unit writes the correction coefficient and the reference light amount value read from the first storage device into the second storage device after the light source module is incorporated into the processor, and
at the time of system activation, compares an activation-time light amount value, which is the reference light amount value of the light emitting device acquired under the predetermined condition, with each of a first light amount value, which is the reference light amount value read from the first storage device, and a second light amount value, which is the reference light amount value read from the second storage device, and determines which of the data stored in the first storage device and the second storage device is more probable data on a basis of a comparison result.

If a difference between the activation-time light amount value and the first light amount value is larger than a predetermined threshold and a difference between the activation-time light amount value and the second light amount value is larger than the predetermined threshold, the control unit may determine that the light source module has failed.

If a difference between the activation-time light amount value and the first light amount value is larger than a predetermined threshold and a difference between the activation-time light amount value and the second light amount value is less than the predetermined threshold, the control unit may determine that the data stored in the second storage device is more probable than the data stored in the first storage device.

In a case where the control unit determines that the data stored in the second storage device is more probable, the control unit may overwrite the data in the first storage device with the data read from the second storage device.

If a difference between the activation-time light amount value and the first light amount value is less than a predetermined threshold and a difference between the activation-time light amount value and the second light amount value is larger than the predetermined threshold, the control unit may determine that the data stored in the first storage device is more probable than the data stored in the second storage device.

In a case where the control unit determines that the data stored in the first storage device is more probable, the control unit may overwrite the data in the second storage device with the data read from the first storage device.

### Advantageous Effects of Invention

According to the above-described electronic endoscopic system, it is possible to ensure robustness of data without using a highly durable storage device in a case where data for correcting an output characteristic of a light source is stored in a storage device in the electronic endoscopic system.

### Brief Description of Drawings

Fig. 1 is a block diagram illustrating an example of a configuration of an electronic endoscopic system according to an embodiment.
Fig. 2 is a block diagram focusing on a portion related to light source control in the electronic endoscopic system according to the embodiment.
Fig. 3 is a diagram illustrating a configuration for calibrating an LED in the electronic endoscopic system according to the embodiment.
Fig. 4 illustrates a relationship between a duty ratio of a PWM signal applied to the LED and illuminance before and after correction.
Fig. 5 is a diagram illustrating an example of data processing on a memory when calibration of a light source module is performed.
Fig. 6 is a diagram illustrating an example of data processing on a memory when the light source module is incorporated into a processor.
Fig. 7 is a flowchart illustrating activation processing of the electronic endoscopic system according to the embodiment.
Fig. 8 is a flowchart illustrating activation processing of the electronic endoscopic system according to the embodiment.
Fig. 9 is a diagram illustrating an example of data processing on the memory when the light source module is replaced.
Fig. 10 is a diagram illustrating an example of data processing on the memory when the memory of the light source module has lost the data.

### Description of Embodiments

Hereinbelow, an electronic endoscopic system according to the present embodiment will be described in detail with reference to the drawings.

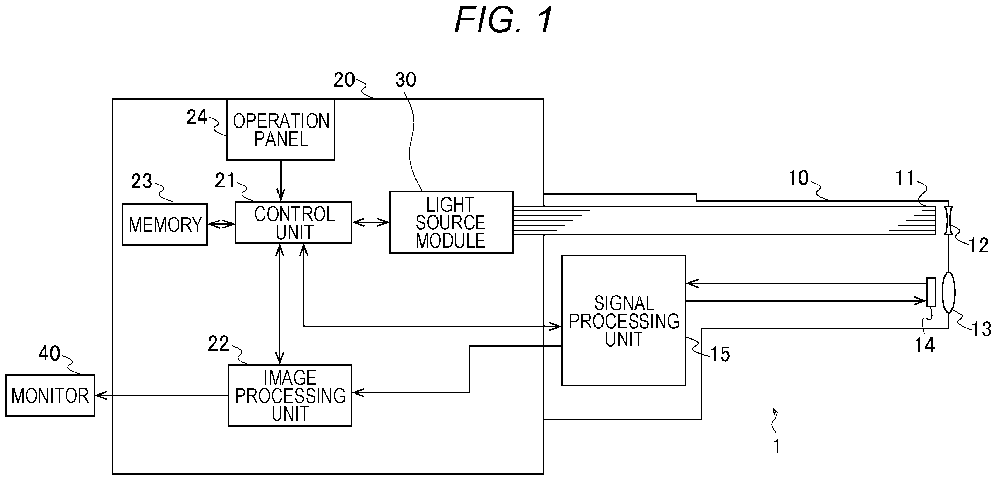

Fig. 1 is a block diagram illustrating an example of a configuration of an electronic endoscopic system 1 of the present embodiment. As illustrated in Fig. 1, the electronic endoscopic system 1 is a system specialized for medical use, and includes an electronic scope (endoscope) 10, a processor for an electronic endoscope 20 (hereinbelow, simply referred to as a "processor 20") to which the electronic scope 10 is connected via a connector, and a monitor 40.

The processor 20 includes a control unit 21. The control unit 21 includes a CPU that executes various programs stored in a memory 23 and integrally controls the entire electronic endoscopic system 1. In addition, the control unit 21 is connected to an operation panel 24. The control unit 21 changes each operation of the electronic endoscopic system 1 and a parameter for each operation in accordance with an instruction from an operator input into the operation panel 24. The control unit 21 supplies a clock pulse serving as a reference for timing of an operation of each unit in the electronic endoscopic system 1 to each unit in the system.

The processor 20 is provided with a light source module 30. The light source module 30 includes a light emitting device (LED) that emits illumination light for illuminating an object such as a body tissue in a body lumen. When one LED is used, the LED is preferably a white LED. Although not illustrated, a plurality of LEDs may be provided. The plurality of LEDs each have a different wavelength band of emitted light, and for example, the plurality of LEDs include a UVLED, a blue LED, a green LED, an amber LED, and a red LED. The illumination light is white light or pseudo white light obtained by combining pieces of light emitted from the plurality of LEDs. The white light is light having a flat spectral intensity distribution in a visible light band, and the pseudo white light is light having a non-flat spectral intensity distribution and obtained by mixing pieces of light of a plurality of wavelength bands.

The illumination light of the light source module 30 is condensed onto an incident end face of a light carrying bundle (LCB) 11 provided in the electronic scope 10, and enters the LCB 11. The illumination light emitted from an emission end face of the LCB 11 is applied to the object via a light distribution lens 12. Return light from the object illuminated with the illumination light from the light distribution lens 12 forms an optical image on a light receiving surface of an image sensor 14 via an objective lens 13.

At the time of manufacturing the light source module 30, calibration for one LED or each of a plurality of LEDs of the light source module 30 is performed as described below. The calibration is processing for ensuring linearity of the LED. As a dimming method of the LED, PWM dimming and analog dimming are known. The PWM dimming is a method of adjusting the illuminance of the LED by adjusting the duty ratio of the PWM signal, and the analog dimming is a method of adjusting the illuminance of the LED by changing the amount of current supplied to the LED. In any of the dimming methods, in the LED before calibration, the relationship of the illuminance of the LED with respect to the input such as the duty ratio of the PWM signal or the current is not linear. Therefore, the purpose of the calibration is to set a correction coefficient for making adjustment so that the relationship is linear.

The light source module 30 is incorporable into a housing (not illustrated) of the processor 20. At the time of manufacturing the light source module 30, the calibration for one LED or each of the plurality of LEDs is performed, and the calibrated light source module 30 is then incorporated into the processor 20. In addition, in a case where the light source module 30 fails or deteriorates over time, a new light source module 30 can be calibrated and then incorporated into the processor 20.

The image sensor 14 is an image sensor having a Bayer pixel arrangement. The image sensor 14 accumulates an optical image formed by each pixel on the light receiving surface as charges corresponding to a light amount and reads the optical image to generate and output imaging data. Note that a CMOS image sensor, a CCD image sensor, or another type of imaging device can be used as the image sensor 14. The image sensor 14 may include a complementary color filter.

The electronic scope 10 includes a signal processing unit 15 in a connection unit to the processor 20.

The signal processing unit 15 performs processing of driving the image sensor 14 and acquires the imaging data from the image sensor 14 under the control of the control unit 21.

In one embodiment, the signal processing unit 15 supplies a synchronization signal (for example, a vertical synchronization signal) of one frame to the image sensor 14, acquires the imaging data of the object from the image sensor 14 per frame, and transmits the imaging data to an image processing unit 22 of the processor 20. A frame cycle is, for example, 1/30 seconds or 1/60 seconds. Note that the present invention is not limited to a case where the signal processing unit 15 supplies the synchronization signal of one frame. Some image sensors transmit the imaging data on the basis of the synchronization signal created by the image sensor itself. In this case, it is not necessary for the signal processing unit 15 to supply the synchronization signal of one frame to the image sensor.

The image processing unit 22 of the processor 20 buffers the imaging data output from the signal processing unit 15 and performs predetermined image processing on the imaging data, then generates a video format signal, and outputs the video format signal to the monitor 40. Examples of the image processing include demosaic processing, matrix calculation, and edge enhancement processing.

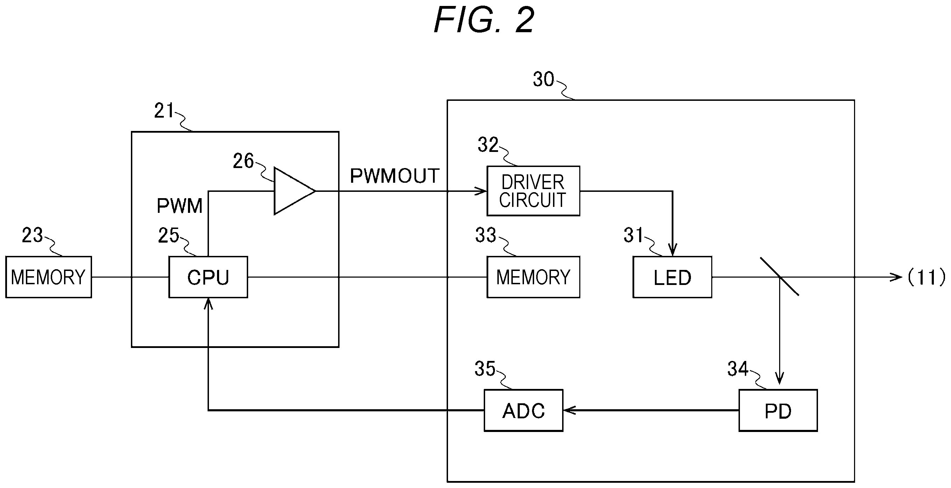

Next, a portion related to light source control in the electronic endoscopic system 1 in Fig. 1 will be described with reference to Fig. 2. Fig. 2 is a block diagram illustrating a portion related to light source control in the electronic endoscopic system 1 in more detail.

Referring to Fig. 2, the light source module 30 includes an LED 31, a driver circuit 32, a memory 33, a photodetector 34, and an analogue to digital converter (ADC) 35.

The control unit 21 includes a CPU 25 and a PWM adjustment unit 26.

As described above, the light source module 30 includes one or a plurality of LEDs. However, since the configuration for performing the processing on each LED is in common, an LED in one system will be described below. For example, in Fig. 2, in the light source module 30, the driver circuit 32 and the LED 31 in one system are illustrated for a signal PWMOUT having a PWM waveform supplied from the control unit 21. In a case where a plurality of LEDs are provided, as many systems as the number of LEDs actually provided are provided in the light source module 30.

In the following description, an example in which the LED uses the PWM dimming method will be described, but the same can be applied to a case of the analog dimming.

The memory 23 (an example of a second storage device) stores a correction coefficient for making the output characteristic of the LED 31 linear. Since the memory 23 is provided in the processor 20, hereinbelow, it is appropriately referred to as a "processor-side memory".

When the processor 20 is activated, the CPU 25 generates and outputs a signal PWM having a PWM waveform for operating the LED 31 of the light source module 30. The duty ratio of the generated signal PWM is a value corresponding to required illuminance based on an operation input into the operation panel 24 or automatic dimming control that is not illustrated. Note that the generation and output of the signal PWM may be executed by an ASIC, an FPGA, or the like that is not illustrated instead of the CPU 25.

The PWM adjustment unit 26 adjusts the duty ratio of the signal PWM supplied from the CPU 25 according to the correction coefficient stored in the memory 23 or the memory 33, and generates the signal PWMOUT having a PWM waveform whose duty ratio has been adjusted (corrected).

The driver circuit 32 drives the LED 31 on the basis of the signal PWMOUT supplied from the control unit 21.

The memory 33 (an example of a first storage device) stores a correction coefficient for making the output characteristic of the LED 31 linear. Since the memory 33 is provided in the light source module 30, hereinbelow, it is appropriately referred to as a "light source-side memory". Data stored in the light source-side memory 33 is readable from the CPU 25. If there is no problem in activation processing to be described below, the same correction coefficient is stored in the light source-side memory 33 and the processor-side memory 23.

The photodetector 34 includes, for example, a photodiode (PD), and outputs an electric signal (analog signal) corresponding to the light intensity of the emitted light of the LED 31. The ADC 35 converts the electric signal output from the photodetector 34 into a digital signal and transmits the digital signal to the CPU 25. As will be described below, the output signal of the photodetector 34 is used in order for the CPU 25 to determine which one of the correction coefficient stored in the light source-side memory 33 and the correction coefficient stored in the processor-side memory 23 is probable.

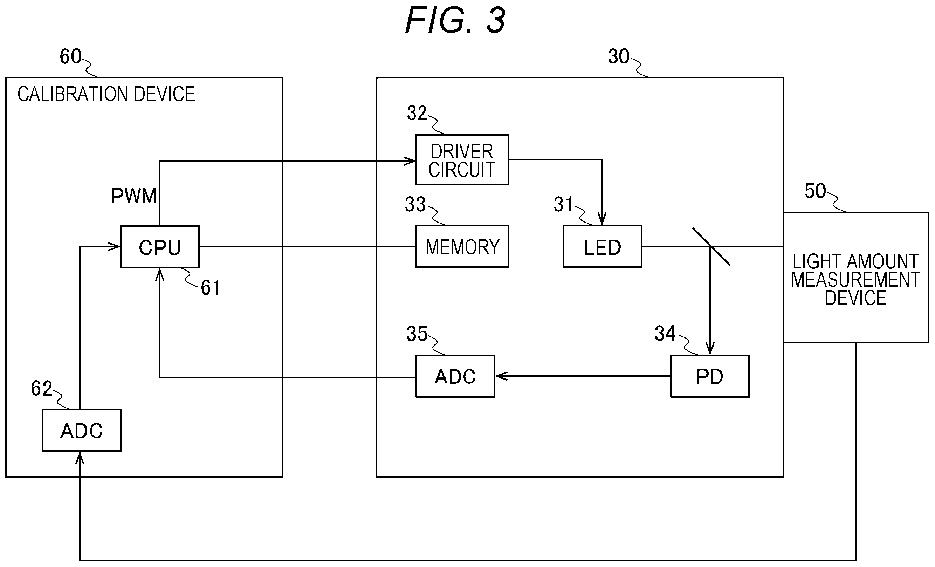

Next, calibration performed at the time of manufacturing the light source module 30 will be described with reference to Figs. 3 to 5. Fig. 3 is a diagram illustrating a configuration for calibrating the LED 31. As described above, at the time of manufacturing the light source module 30, calibration for one LED or each of a plurality of LEDs of the light source module 30 is performed.

At the time of performing calibration, as illustrated in Fig. 3, a light amount measurement device 50 and a calibration device 60 are connected to the light source module 30 as a calibration target.

The light amount measurement device 50 acquires the emitted light of the LED 31 and supplies an electric signal (analog signal) corresponding to the light amount (or illuminance) of the emitted light to the calibration device 60.

The calibration device 60 includes a CPU 61 and an ADC 62. The CPU 61 is configured to execute a predetermined calibration program. By executing the calibration program, the CPU 61 generates the signal PWM whose PWM waveform has different duty ratios from each other and supplies the signal to the driver circuit 32. The CPU 61 acquires the output (corresponding to the illuminance of the LED 31) of the photodetector 34 corresponding to each of the different duty ratios of the signal PWM, and calculates a correction coefficient to be described below.

The ADC 62 converts an electric signal supplied from the light amount measurement device 50 into a digital signal and transmits the digital signal to the CPU 61. An electric signal output from the photodetector 34 of the light source module 30 is converted into a digital signal by the ADC 35 and transmitted to the CPU 61.

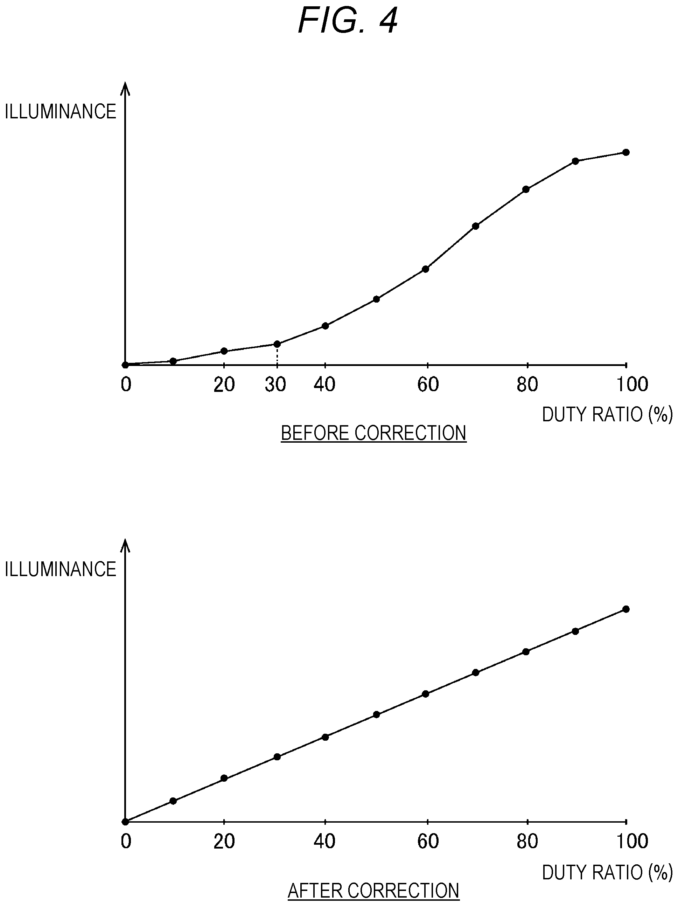

As an example, Fig. 4 illustrates an example of the relationship between the duty ratio of the signal PWM and the illuminance (illuminance at a position away from the LED by a predetermined distance) in the LED before correction. Before the correction, the relationship between the duty ratio and the illuminance of the LED is nonlinear. In the calibration, the relationship between the duty ratio of the signal PWM and the illuminance of the LED before the correction is acquired, and the relationship between the duty ratio of the signal PWM and the duty ratio of the signal PWMOUT is determined so that the relationship between the duty ratio of the signal PWMOUT and the illuminance of the LED becomes linear.

Here, in a case where the duty ratio of the signal PWM is x and the duty ratio of the signal PWMOUT is y, the CPU 61 executes a predetermined calibration program to calculate correction coefficients C0 to C6 of a polynomial model represented by the following equation (1) so that the relationship between the duty ratio of the signal PWMOUT and the illuminance of the LED becomes linear. $y = C 6 ⋅ {x}^{6} + C 5 ⋅ {x}^{5} + C 4 ⋅ {x}^{4} + C 3 ⋅ {x}^{3} + C 2 ⋅ {x}^{2} + C 1 ⋅ x + C 0$ (1)

Note that the polynomial model is an example, and any model can be applied. The calibration program calculates a correction coefficient for ensuring linearity of the LED using the polynomial model or another model.

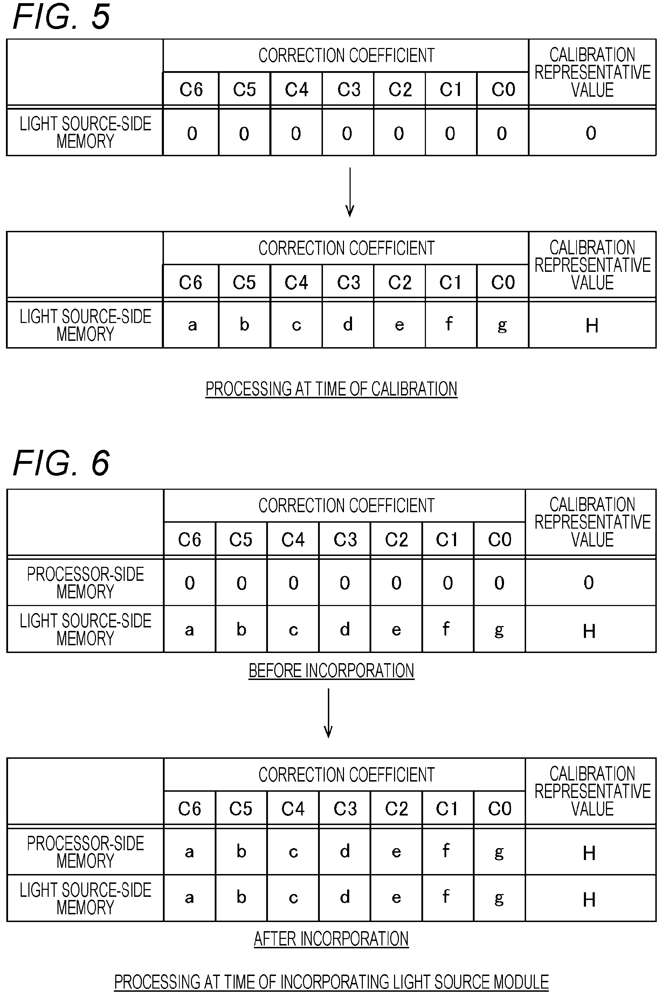

The CPU 61 of the calibration device 60 executes the calibration program to calculate the correction coefficients C0 to C6, and then writes the calculated correction coefficients in the light source-side memory 33 of the light source module 30 as illustrated in Fig. 5. In the example illustrated in Fig. 5, a case is illustrated where the correction coefficients recorded in the light source-side memory 33 before writing are all "0", and the correction coefficients C6, C5, C4, C3, C2, C1, and C0 to be written are a, b, c, d, e, f, and g, respectively.

In addition, the CPU 61 writes an output signal (digital value; this is referred to as "PD output") of the photodetector 34 under a predetermined condition in the light source-side memory 33. The PD output under the predetermined condition is referred to as a "calibration representative value" (an example of a reference light amount value). Although the "predetermined condition" is not limited to a particular one, a case where the PD output is acquired when the duty ratio of the signal PWM supplied by the CPU 61 is 30% will be described below. The "predetermined condition" may be any duty ratio as long as the condition is not changed according to the time point (for example, the time of executing calibration, the time of activating the system, the time of operating the system, and the like) at which the PD output is acquired. In other words, the "predetermined condition" at the time of acquiring the PD output is always the same condition.

Here, the calibration representative value written in the light source-side memory 33 is compared with the PD output acquired under the same condition when the electronic endoscopic system 1 is activated, and is used for failure determination.

The calibrated light source module 30 is incorporated into the processor 20.

At the first activation of the electronic endoscopic system 1 after the light source module 30 is incorporated into the processor 20, the control unit 21 of the processor 20 writes the correction coefficient and the calibration representative value recorded in the light source-side memory 33 into the processor-side memory 23.

Fig. 6 illustrates examples of the correction coefficients and the calibration representative values recorded in the processor-side memory 23 and the light source-side memory 33 before and after the light source module 30 is incorporated into the processor 20.

Before the incorporation, the values recorded in the light source-side memory 33 are the values after execution of the calibration illustrated in Fig. 5. Before the incorporation, initial values (here, all "0") at the time of factory shipment are recorded in the processor-side memory 23. When the light source module 30 is incorporated into the processor 20, as illustrated in Fig. 6, the same correction coefficients and the same calibration representative values are recorded in the processor-side memory 23 and the light source-side memory 33.

Preferably, after the data is written, the data recorded in the processor-side memory 23 is read, and the read data is verified. For example, if the value read from the processor-side memory 23 does not match the value read from the light source-side memory 33, it is determined that the verification has failed, and it is determined that the processor-side memory 23 has failed.

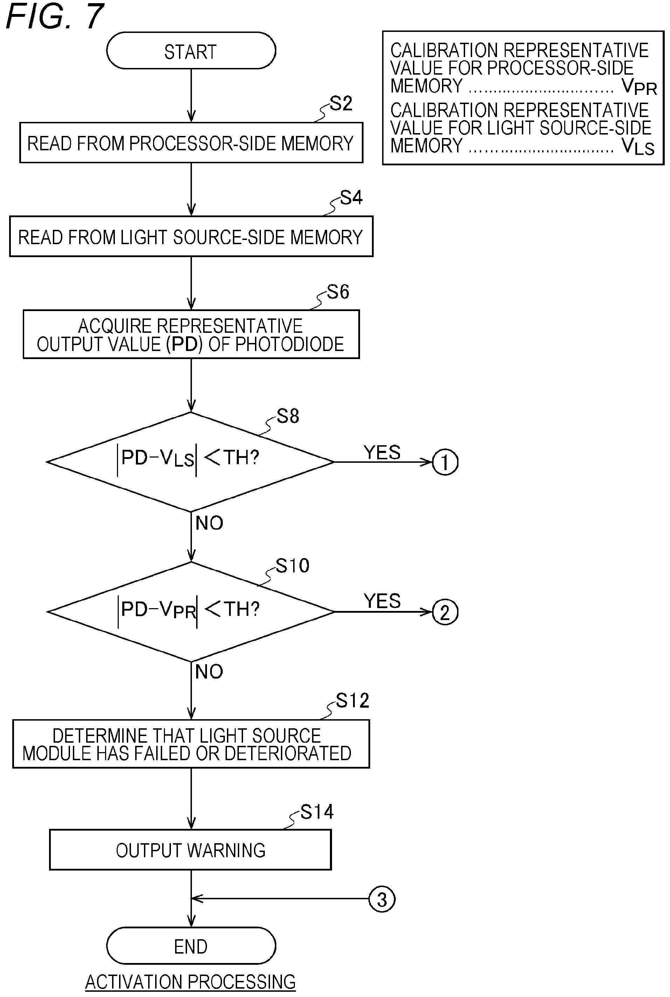

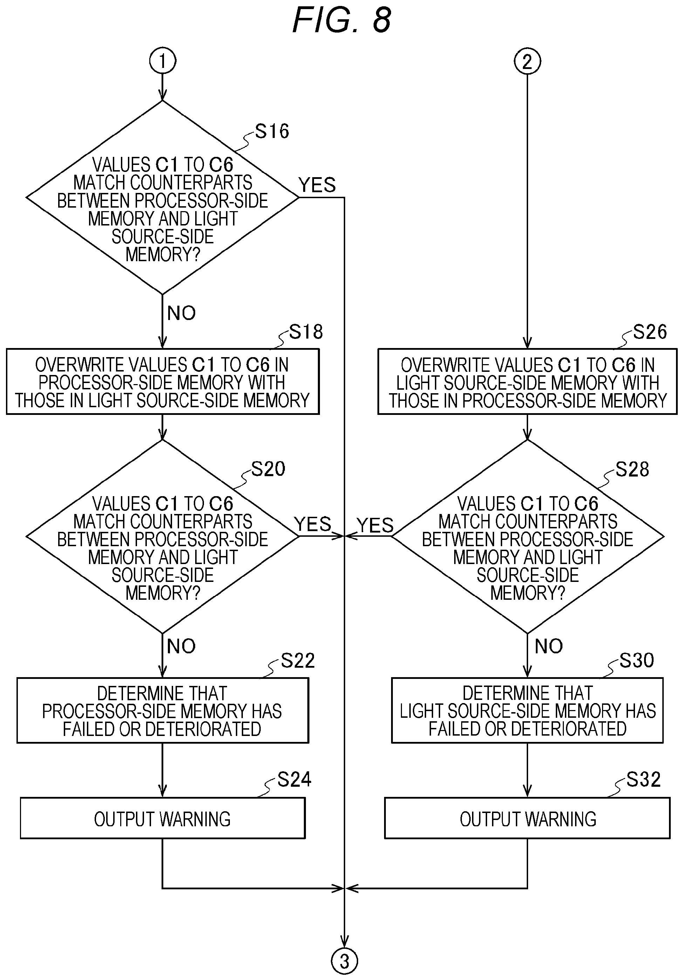

Next, activation processing of the electronic endoscopic system 1 will be described with reference to flowcharts in Figs. 7 and 8.

Note that the activation processing illustrated in Figs. 7 and 8 assumes that the same correction coefficient and the same calibration representative value are recorded in the processor-side memory 23 and the light source-side memory 33 in the initial activation of the electronic endoscopic system 1, and means each time of activation processing thereafter. The activation processing illustrated in Figs. 7 and 8 is executed by the processor 20 (mainly, the control unit 21 of the processor 20).

In the activation processing, first, the processor 20 sequentially reads the correction coefficients and the calibration representative values recorded in the processor-side memory 23 and the light source-side memory 33 (steps S2 and S4). Here, the calibration representative values read from the processor-side memory 23 and the light source-side memory 33 are examples of a second light amount value and a first light amount value, respectively.

Subsequently, the processor 20 acquires a PD representative output value (PD output) (expressed as "PD" in the flowchart; an example of an activation-time light amount value) of the photodetector 34 derived when the signal PWM with the duty ratio of 30% (the same condition as when the calibration representative value is acquired) is output from the control unit 21 (step S6).

Note that, when the PD output is acquired, the duty ratio is not adjusted by the PWM adjustment unit 26 (Fig. 2). That is, a signal PWM generated by the control unit 21 is supplied to the light source module 30 as it is. By doing so, the PD output is acquired under the same condition as when the calibration representative value is acquired at the time of executing the calibration.

The control unit 21 compares the PD output acquired in step S6 with a calibration representative value V_{LS} read from the light source-side memory 33, and determines whether or not a difference between the PD output and the calibration representative value V_{LS} is less than a predetermined threshold TH (step S8). If the difference between the two is less than the threshold TH (step S8: YES), that means that the PD output is substantially the same as the calibration representative value acquired when the calibration is executed for the light source module 30, and thus, it is considered that the data of the light source-side memory 33 is highly reliable. In this case, the correction coefficients C0 to C6 are also read from the light source-side memory 33 and the processor-side memory 23, and it is determined whether the correction coefficients C0 to C6 all match the counterparts. If they match (step S16: YES), that means that both the light source-side memory 33 and the processor-side memory 23 are considered to be normal, and thus the activation processing ends.

Conversely, if at least one of the correction coefficients C0 to C6 does not match the counterpart between the light source-side memory 33 and the processor-side memory 23 (step S16: NO), this means that the data value in the processor-side memory 23 is different from the data value in the light source-side memory 33, which has been considered to be highly reliable in step S8. In this case, the control unit 21 determines that the data stored in the light source-side memory 33 is more probable than the data stored in the processor-side memory 23. Furthermore, the control unit 21 overwrites the correction coefficients C0 to C6 in the processor-side memory 23 with those in the light source-side memory 33 (step S18).

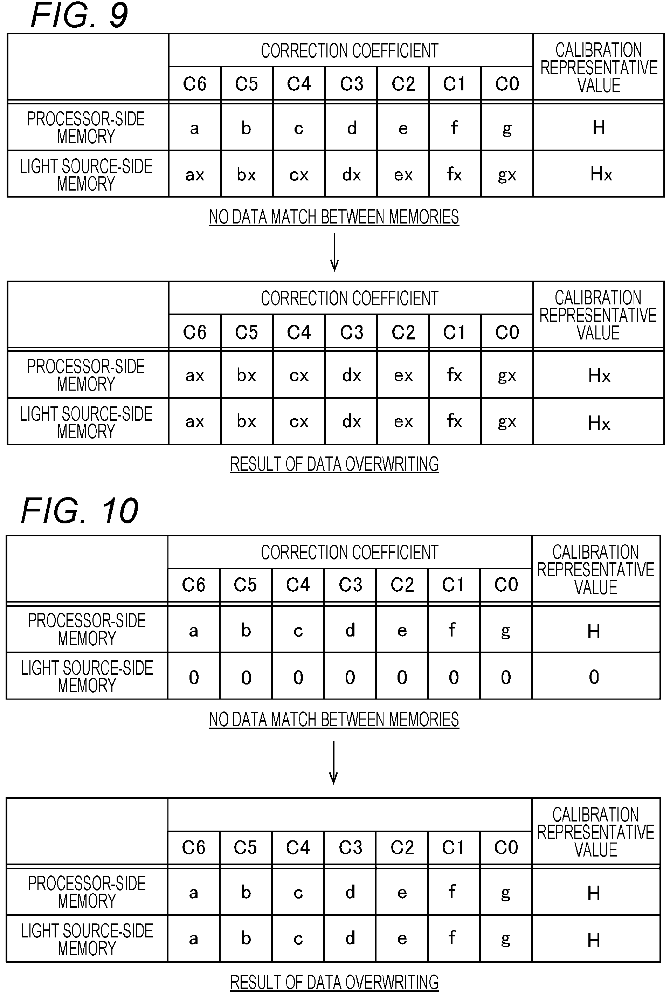

Fig. 9 illustrates an example of a case where the data does not match between the light source-side memory 33 and the processor-side memory 23. In this example, the data in the light source-side memory 33 is changed to ax, bx, cx, dx, ex, fx, and gx from the state at the time of initial system activation (the state after incorporation in Fig. 6), and thus, the data does not match between the memories. In this case, the data in the processor-side memory 23 becomes ax, bx, cx, dx, ex, fx, and gx by the data overwriting in step S18 to make the data match between the memories.

If it is confirmed as a result of the overwriting in step S18 that all the values of the correction coefficients C0 to C6 match the counterparts between the processor-side memory 23 and the light source-side memory 33 (step S20: YES), that means that the data in the processor-side memory 23 is restored, and the activation processing thus ends.

If the values of the correction coefficients C0 to C6 do not all match the counterparts between the processor-side memory 23 and the light source-side memory 33 even by the overwriting in step S18 (step S20: NO), the control unit 21 determines that the processor-side memory 23 has failed or deteriorated (step S22), outputs a warning to the monitor 40 or the like (step S24), and ends the activation processing.

If, as a result of comparing the PD output acquired in step S6 with the calibration representative value V_{LS} read from the light source-side memory 33, a difference between the PD output and the calibration representative value V_{LS} is equal to or more than the predetermined threshold TH (step S8: NO), the control unit 21 can determine that the light source-side memory 33 may have failed or deteriorated. In this case, the processing proceeds to step S10, where the PD output acquired in step S6 is compared with the calibration representative value V_{PR} read from the processor-side memory 23, and it is determined whether or not a difference between the PD output and the calibration representative value V_{PR} is less than the predetermined threshold TH.

If the difference between the two is equal to or more than the threshold TH in step S10 (step S10: NO), that means that only the PD output, among the calibration representative value V_{PR} read from the processor-side memory 23, the calibration representative value V_{LS} read from the light source-side memory 33, and the PD output, differs from the other two calibration representative values, and thus the light source module 30 has highly possibly failed or deteriorated. Therefore, the control unit 21 determines that the light source module 30 has failed or deteriorated (step S12), outputs a warning to the monitor 40 or the like (step S14), and ends the activation processing.

Note that it is considered that the failure or deterioration of the light source module 30 is caused by a failure of a transistor element of the driver circuit 32, a failure or deterioration of the LED 31, a failure or deterioration of the photodetector 34, a failure of the ADC 35, an optical path axis deviation of the emitted light of the LED 31, deterioration of the optical path due to the ultraviolet light, and the like.

If the difference between the two is less than the threshold TH in step S10 (step S10: YES), that means that the PD output is substantially the same as the calibration representative value written at the time of the first activation after the light source module 30 is incorporated into the processor 20, and thus it is considered that the data in the processor-side memory 23 is highly reliable. That is, since only the calibration representative value V_{LS} read from the light source-side memory 33, among the calibration representative value V_{PR} read from the processor-side memory 23, the calibration representative value V_{LS} read from the light source-side memory 33, and the PD output, differs from the other two values, the light source module 30 has highly possibly failed or deteriorated.

In this case, the control unit 21 determines that the data stored in the processor-side memory 23 is more probable than the data stored in the light source-side memory 33. Furthermore, the control unit 21 overwrites the correction coefficients C0 to C6 in the light source-side memory 33 with those in the processor-side memory 23 (step S26).

Fig. 10 illustrates an example of a case where the data does not match between the memories because the light source-side memory 33 has lost the data. In this example, the light source-side memory 33 has lost the data from the state at the time of initial system activation (the state after incorporation in Fig. 6), and all the correction coefficients C0 to C6 are 0. In this case, the data in the light source-side memory 33 becomes a, b, c, d, e, f, and g by the data overwriting in step S26 to make the data match between the memories.

If it is confirmed as a result of the overwriting in step S26 that all the values of the correction coefficients C0 to C6 match the counterparts between the processor-side memory 23 and the light source-side memory 33 (step S28: YES), that means that the data in the light source-side memory 33 is restored, and the activation processing thus ends.

If the values of the correction coefficients C0 to C6 do not all match the counterparts between the processor-side memory 23 and the light source-side memory 33 even by the overwriting in step S26 (step S28: NO), the control unit 21 determines that the light source-side memory 33 has failed or deteriorated (step S30), outputs a warning to the monitor 40 or the like (step S32), and ends the activation processing.

As described above, in the electronic endoscopic system 1 according to the embodiment, after the light source module 30 is incorporated into the processor 20, the control unit 21 of the processor 20 writes the correction coefficient and the calibration representative value read from the light source-side memory 33 of the light source module 30 into the processor-side memory 23.

Furthermore, at the time of system activation, as illustrated in Figs. 7 and 8, the control unit 21 compares the PD output of the LED 31 acquired under a predetermined condition with the calibration representative value read from the light source-side memory 33 and with the calibration representative value read from the processor-side memory 23, and determines which of the data stored in the light source-side memory 33 and the processor-side memory 23 is more probable data on the basis of the comparison result. Therefore, the following effects are exhibited.

In a case where the data for correcting the output characteristic of the LED is stored in the memory, a redundant configuration including the processor-side memory and the light source-side memory is adopted, and the PD output of the photodetector sequentially acquired from the processor is compared with each of the data read from the processor-side memory and the data read from the light source-side memory to monitor both the data. Therefore, the robustness of the data can be ensured without using a highly durable memory.

By using the photodetector that detects the emitted light of the LED provided in the processor, it is easy to identify which of the processor-side memory, the light source-side memory, and the components other than the light source-side memory in the light source module has failed or deteriorated.

As described above, calibration using the light amount measurement device and the calibration device is performed at the time of manufacturing the light source module, and the correction coefficient and the like are written in the light source-side memory. When the light source module is incorporated into the processor, the written correction coefficient and the like are copied and written in the processor-side memory. Therefore, at the time of incorporating the light source module into the processor, recalibration is not necessary, and thus the manufacturing process can be simplified.

The electronic endoscopic system of the present invention has been described above in detail, but the electronic endoscopic system of the present invention is not limited to the above embodiment. As a matter of course, various improvements or modifications may be made within a scope not departing from the concept of the present invention.

The present invention is related to a patent application of Japanese Patent Application No. 2023-210077 filed with the Japan Patent Office on December 13, 2023, the entire contents of which are incorporated herein by reference.

## Claims

1. An electronic endoscopic system configured to perform imaging of a body tissue, the electronic endoscopic system comprising:
a processor that performs processing of a captured image of the body tissue; and
a light source module that is incorporable into the processor for an electronic endoscope and generates illumination light for the body tissue on a basis of light emitted from at least one light emitting device,
wherein the light source module includes
a first storage device that stores a correction coefficient for correcting an output characteristic of the light emitting device and a reference light amount value of the light emitting device under a predetermined condition, the correction coefficient and the reference light amount value being obtained before the light source module is incorporated into the processor,
wherein the processor includes
a second storage device, a photodetector that detects a light amount of the light emitting device, and a control unit, and
wherein the control unit
writes the correction coefficient and the reference light amount value read from the first storage device into the second storage device after the light source module is incorporated into the processor, and
at time of system activation, compares an activation-time light amount value, which is the reference light amount value of the light emitting device acquired under the predetermined condition, with each of a first light amount value, which is the reference light amount value read from the first storage device, and a second light amount value, which is the reference light amount value read from the second storage device, and determines which of the data stored in the first storage device and the second storage device is more probable data on a basis of a comparison result.

2. The electronic endoscopic system according to claim 1,
wherein, if a difference between the activation-time light amount value and the first light amount value is larger than a predetermined threshold and a difference between the activation-time light amount value and the second light amount value is larger than the predetermined threshold, the control unit determines that the light source module has failed.

3. The electronic endoscopic system according to claim 1,
wherein, if a difference between the activation-time light amount value and the first light amount value is larger than a predetermined threshold and a difference between the activation-time light amount value and the second light amount value is less than the predetermined threshold, the control unit determines that the data stored in the second storage device is more probable than the data stored in the first storage device.

4. The electronic endoscopic system according to claim 3,
wherein, in a case where the control unit determines that the data stored in the second storage device is more probable, the control unit overwrites the data in the first storage device with the data read from the second storage device.

5. The electronic endoscopic system according to claim 1,
wherein, if a difference between the activation-time light amount value and the first light amount value is less than a predetermined threshold and a difference between the activation-time light amount value and the second light amount value is larger than the predetermined threshold, the control unit determines that the data stored in the first storage device is more probable than the data stored in the second storage device.

6. The electronic endoscopic system according to claim 5,
wherein, in a case where the control unit determines that the data stored in the first storage device is more probable, the control unit overwrites the data in the second storage device with the data read from the first storage device.
